# EUROPEAN PATENT APPLICATION

(11) **EP 3 340 380 A1**
(43) Date of publication of application: **27.06.2018**
(21) Application number: 16205559.4
(22) Date of filing: 20.12.2016
(51) Int. Cl.: H01Q 1/40, A61B 5/00

(54) **DEVICE FOR ARRANGEMENT UNDERNEATH ONE OR MULTIPLE LAYERS OF MATERIAL**

(71) Applicant: Vestel Elektronik Sanayi ve Ticaret A.S., 45030 Manisa (TR)
(72) Inventor: MEMISOGLU, Görkem, 45030 Manisa (TR); ABBAK, Mehmet, 45030 Manisa (TR); ÖCAL, Ömür, 45030 Manisa (TR)
(74) Representative: Ascherl, Andreas

(57) **Abstract**

The present invention refers to a device (1) for arrangement underneath one or multiple layers (2, 4, 6) of material. Said device comprises at least
a detection unit (8) for detecting of at least one parameter of the material,
a communication unit (10) for transmitting data representing the detected parameter through the one or multiple layers (2, 4, 6) of material,
wherein the communication unit (10) comprises a RF circuit (12) and an antenna (14),
an envelope element (16) for surrounding of at least the communication unit (10),
wherein the envelope unit (16) comprises a material composition, wherein the material composition at least comprises a polymer material, a ceramic material and cellulose.

## Description

The present invention refers according to claim 1 to a device for arrangement underneath one or multiple layers of material, according to claim 8 to a method for manufacturing of such a device, according to claim 9 to a method for supervising of substance or material properties, in particular vital parameters of a living being, and according to claim 14 to a wireless mesh network for supervising of an agriculture system.

Prior art document US2014257052A1 discloses an antenna which is placed just under the skin. This system is encapsulated to conserve the structure from the corrosion and ensure corrosion resistance.

Document US2008180242A1 discloses, that ferrite has been used to enhance the magnetic permeability of the antenna. A near-field non-radiating magnetic coupling technique is used which is very limited in terms of information exchange, compared to the electromagnetic coupling, in which electromagnetic waves propagate.

The known antenna systems do not provide homogenous data transmission through the adjacent material respectively material layers.

Thus, it is the object of the present invention to provide a device and a production method for such a device that enables improved data transmission compared to the known techniques.

The before mentioned object is solved by a device according to claim 1 for arrangement underneath one or multiple layers of material. The inventive device preferably comprises at least a detection unit for detecting of at least one parameter of the material, a communication unit for transmitting data representing the detected parameter through the one or multiple layers of material, wherein the communication unit preferably comprises a wireless communication means, in particular a RF circuit, and an antenna, an envelope element for surrounding the detection unit and/or the communication unit, wherein the envelope unit comprises a material composition, wherein the material composition at least comprises a polymer material, a ceramic material and cellulose, wherein the ratio (in wt%) of these materials is selected in such a manner to set up a predefined dielectric property.

This solution is beneficial, since the main advantage of this material composite is that different dielectric properties can be set up respectively provided according to the location inside the human body.

Any electromagnetic wave travels in a medium, which defines the parameters of the wave propagation. When the transmit and receive antennas are in the same medium no separate effect can be observed due to stable aspect of the medium. However, when the antennas are in the different mediums, some part of the electromagnetic wave is reflected and some part is transmitted to the other medium of propagation. This ratio of the transmission from one medium to another is related to dielectric properties of the medium. When the difference between the different mediums is so high reflection at the boundary is very high too. That is because that, the refection ratio is proportional to the distinction of two mediums. Electromagnetic waves confront to the back reflection when the medium, in which antenna presents, changes. With the increase in the ratio of transition between the medium where electromagnetic waves propagate, more wave reflection occurs at the boundary. The dielectric constant of the air, where the receiver device is going to be outside of the body, is close to the εr = 1. Whereas, in the human body different types of the tissues with different dielectric properties exist.

By inserting intermediary step mediums to minimize the radical distinctions much more of the electromagnetic wave can be transmitted between the different mediums. Correspondingly, by encapsulating the device respectively antenna with material that has proper dielectric properties, maximum efficient radiation can be achieved. This mechanism is not related with the type of the device respectively antenna, so, any type of wireless communication device respectively antenna can be applied within this invention. This is also true for the RF circuit and the frequency, which can affect the dielectric properties of the medium.

Thus, electromagnetic matching of the antenna structure to the human body environment or any other desired environment, like soil, is implemented.

In term of usage as implanted devices, this invention provides high efficiency according to the location inside the human body. Therefore, this device respectively antenna provides robust communication e.g. in medical real-time health monitoring devices/systems.

Further preferred embodiments are subject matter of the dependent claims and/or of the following specification passages.

According to a preferred embodiment of the present invention the material composition comprises in wt% more polymer material than the sum of wt% of ceramic material and cellulose. Additionally or alternatively the material composition comprises in wt% more ceramic material than cellulose. These ratios are beneficial since the electric properties of the envelope can be set-up very precise

The material composition comprises according to a further preferred embodiment of the present invention additionally or alternatively to one or more materials (polymer, ceramic, cellulose) at least dye molecules and/or rubber materials. Preferred dye molecules are e.g. ruthenium and/or perylen and a preferred rubber material is NTN.

The material composition comprises according to a further preferred embodiment of the present invention additionally or alternatively Ceramic materials like Rogers RO 3203, Rogers RO 3206, Rogers RO 3210 etc.

The material composition comprises according to a further preferred embodiment of the present invention additionally or alternatively Polymer composite material like PDMS-MCT composites, PDMS-D270 composites etc.

The material composition comprises according to a further preferred embodiment of the present invention additionally or alternatively Celluluse material like Celluluse nanofibers, celluluse nanowhiskers etc.

By the using of polymer composite materials, mixture, also antenna can be flexible, low sensitive to high temperature variations, etc.

Cellulose can be used to fabricate "smart materials". Nanofiber or nanowhisker celluloses can be used in the antenna system. It is proposed to use it as a mixture with polymer and ceramic composition. However, using cellulusic structures for increasing the antenna durableness while decreasing the wrinkless at the antenna surface is also possible.

The before mentioned particles are preferably provided and used in nm or *µ*m scales. There are many options to have them in these scales (such as ball milling, ultrasonicator,...).

Thus, ceramic material and /or cellulose is/are provided according to a further preferred embodiment of the present invention as particles having a size between 0,1nm and 10*µ*m, in particular between 1 nm and 1*µ*m.

The ceramic material and/or cellulose are provided according to a further preferred embodiment of the present invention as powder and is preferably mixed in particularly homogenously. The ceramic material and cellulose and/or any other herein disclosed material are preferably capsuled by the polymer material.

According to the antenna sizes, there can be totally about 100mg - 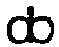 mg materials. Especially for biomedical antennas, antenna size can be nanoantenna. However, for other puposes, antenna can be bigger than nm sizes. Total size is related to the operation frequency of the system. Size of the antenna is approximately quarter wavelength in the medium. For the efficient radiation distance to the outside wall, preferably one tenth of the wavelength is required in all directions.

For example: Considering a telemetry system working at operation frequency 2.4GHz. In a polymer with a dielectric constant of 40, length of the antenna is going to be around 4.9mm. Also, the radius of the composite is going to be 2mm. So, totally 83.4 mm^3 composite is needed to efficiently cover the antenna.

The material composition forms according to a further preferred embodiment at least two layers, wherein one layer preferably comprises polymer material respectively consists of polymer material and wherein another layer preferably comprises more than one material, in particularly two or more than two materials, in particular consists of the ceramic material and cellulose. The ceramic material and cellulose and/or any further material, in particular herein described, are arranged inside a viscose carrier material, in particular a further polymer material. It is conceivable that the viscose carrier material is the same material as the material of the first layer. This embodiment is beneficial since the material composition of the polymer:[Ceramic: x wt% Cellulose] can provide corrosion resistance with the protection of the upper most polymer layer. This layer can also provide the elasticity to the powder mixture of ceramic and cellulose. By providing appropriate ceramic cellulose ratio differently the desired dielectric property is obtained around the antenna for maximum electromagnetic wave transmission.

The before mentioned object is also solved by a method for manufacturing of a before described inventive device. Such a method is provided in claim 9. Said method preferably comprises at least the steps: Providing a detection unit for detecting of at least one parameter of the material and providing a communication unit, in particular a wireless communication unit, for transmitting data representing the detected parameter through the one or multiple layers of material, wherein the communication unit preferably comprises a RF circuit and highly preferable at least one antenna. The method preferably further comprises the step forming an envelope element for surrounding the detection unit and the communication unit, wherein the envelope unit comprises a material composition, wherein the material composition at least comprises a polymer material, a ceramic material and cellulose, wherein the ratio (in wt%) of these materials is selected in such a manner to set up a predefined dielectric property.

Thus, this invention preferably comprises at least a detection and/or communication device that comprises respectively consists of composite material and at least one antenna. Together with the antenna, any RF circuit, regarding to communication of any type of sensor, can be implemented. Therefore, antenna or device efficiency directly effects the robust communication of RF circuit with the outside of the human body. RF circuit can provide real-time data related to any type of sensor (blood pressure, heart rate, body temperature, glucose level, etc.) measuring preferably predefined parameters, in particular physiological specifications. Therefore, the detection unit is preferably equipped to detect vital functions and/or composition changes of gaseous or liquid or solid substances inside the living being.

The before mentioned object is also solved by a method for supervising of substance or material properties. Such a method is provided in claim 10. This inventive method preferably comprises at least the steps: arranging a device according to the present invention underneath a predefined material, wherein the predefined material and the envelope unit are having at least one matching dielectric property, detecting of at least one parameter with the detection unit and transmitting data representing the detected parameter through the material layer or material layers receiving said data by means of a receiving means.

This invention can be applied where at least one device respectively antenna is implanted in a body, in particular organic material, or a further material, in particular not organic material. Medical wireless telemetry and monitoring systems are the main application of the implanted antennas. Besides that, large number of this antenna can be inserted in soil to establish wireless mesh network over large area of field to real time monitoring of crop. With the together usage of the sensors like temperature, humidity more reliable data to wireless mesh network of precision agriculture systems can be provided with implantable antenna. Thus, the detection unit is preferably equipped to detect composition changes of gaseous or liquid or solid substances inside the soil, in particular temperature and/or humidity and/or nutrient content.

Thus, it is conceivable that the inventive device is implanted inside a living being or that the inventive device is arranged inside soil.

According to a further preferred embodiment of the present invention the operating frequency of the RF-circuit is set-up in dependency of material properties of the material layer or material layers through which the data has to be transmitted. This, besides material property differences between the device and the material respectively material layer or material layers the frequency is another factor that specifies the electrical properties of the materials or the tissues. So, with the change in the operating frequency of the system, electrical properties of the medium, in which antennas are implanted changes. According to these discrepancies related to the frequency and tissue properties, antenna can be accurately optimized for efficient radiation characteristics.

To obtain this feature a matching medium with variable dielectric property is needed. The invented device respectively antenna with encapsulating composite material can provide this feature. Also, corrosion protection of the RF circuit and the antenna is provided within the polymer structure.

The present invention can also refer to a wireless mesh network for supervising of an agriculture system. Such a mesh network preferably comprises at least a plurality of devices according to claims 1 to 7 and at least one receiving means for receiving transmitted data. The inventive wireless mesh network preferably also comprises one or more actuator means, in particular a watering system, wherein said actuator means are coupled, in particular in terms of signal exchange, with said mesh network, for treating said agricultural system, in particular watering, in dependency of the received data.

Further benefits, goals and features of the present invention will be described by the following specification of the attached figures, in which exemplarily components of the invention are illustrated. Components of the systems and methods according to the inventions, which match at least essentially with respect to their function can be marked with the same reference sign, wherein such components do not have to be marked or described in all figures.

In the following the invention is just exemplarily described with respect to the attached and explained figures.

### Brief Description of the Drawings

- Fig. 1: shows schematically an antenna device according to the present invention;
- Fig. 2: shows schematically a human being having at least and preferably more than one antenna device implanted;
- Fig. 3: shows multiple layers of soil, wherein at least one antenna device according to the present invention is arranged inside or below one of said layers; and
- Fig. 4: shows schematically a mesh network set-up in agricultural environment.

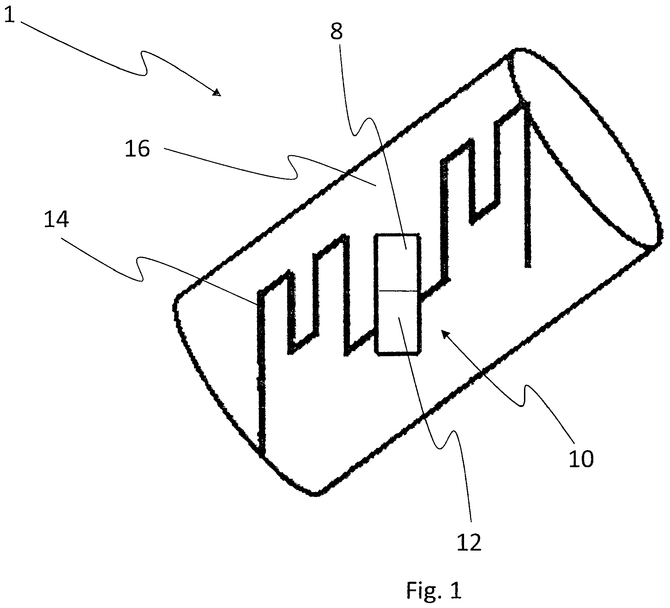

According to fig. 1 the inventive device 1 comprises an envelope unit 16. That envelope unit 16 comprises a material composition, wherein the material composition at least comprises a polymer material, a ceramic material and cellulose. The envelope unit 16 at least sectionally and preferably fully surrounds a detection unit 8, in particular a sensor means for detection material parameters and/or environment parameters and/or health parameters and/or vital parameters. The type of detection means 8 depends on field of application of device 1.

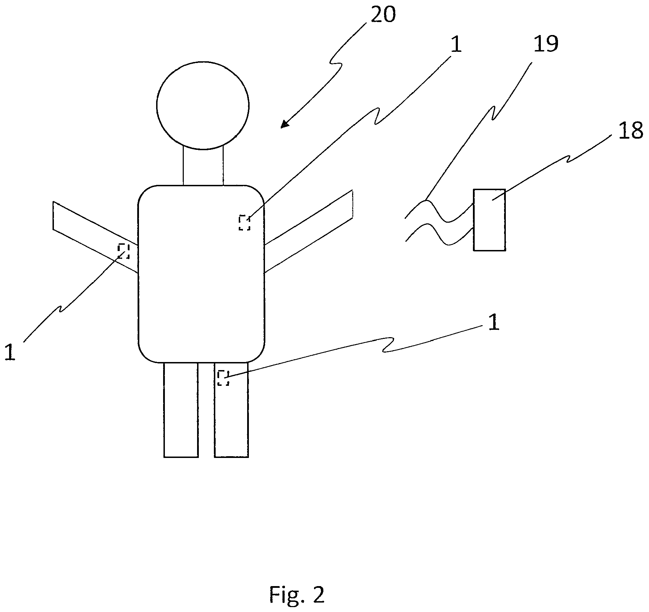

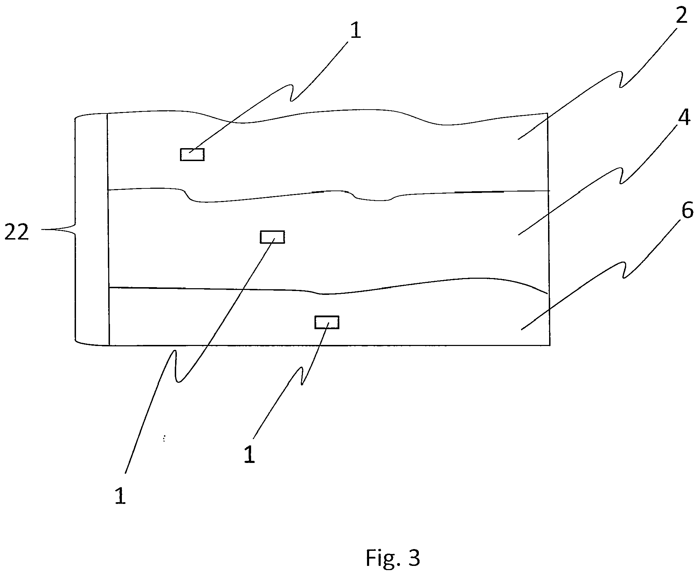

Device 1 further comprises at least one communication unit 10, in particular a RF circuit 12. The communication unit 10 enables data transfer trough a material or through multiple material layers. Thus, data can be exchanged without a haptic or visual connection between device 1 and a receiving means 18 (cf. fig. 2 or fig. 3). The communication unit 10 preferably comprises an antenna 14 for receiving and/or transmitting data and/or energy. Device 1 further preferably comprises an energy storage means (not shown) for providing energy to detection unit 8. The antenna is position-independent. Mixture should be well mixed and coated on antenna substrate. It is preferably not important which side is at the top. For efficient radiation and to minimize the size of the encapsulation, antenna is preferably placed in the centre in all directions.

Fig. 2 shows a scenario in which device 1 is implanted in a living being 20, in particular inside a human body. In dependency of the respective place of implantation the dielectric properties of the material between the antenna 14 and the outside of the human body differ. Thus, the composition of polymer material, ceramic material and cellulose is set up for each position with different ratios. Data can be read out from device 1 by means of a receiving means 18, in particular a NFC device. It is further conceivable that data can be sent to device 1 and said data can be computed respectively processed by device 1. Thus, data exchange between device 1 and receiving means 18 is preferably carried out via electromagnetic waves 19.

Fig. 3 shows a scenario in which device 1 is arranged inside soil 22, in particular in agricultural usage. Device 1 can hereby be arranged in every specific layer 2, 4, 6, wherein the material composition of envelope unit 16 is set up in dependency of the dielectric properties of the respective layer 2, 4, 6.

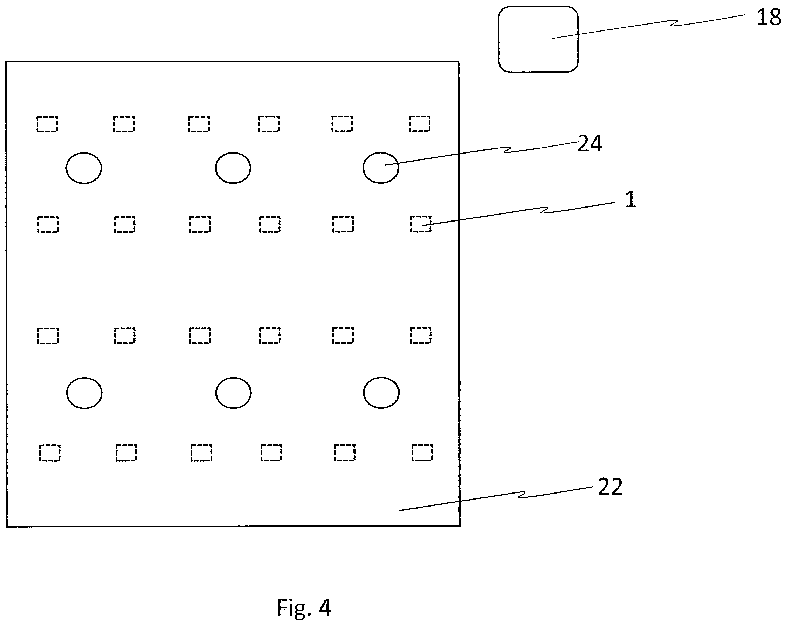

Fig. 4 shows a field, wherein multiple devices 1 are arranged inside the ground of said field. Said devices 1 preferably belong to a mesh network respectively are setting up such a mesh network. The network preferably comprises information about the specific properties of each device 1 and of the location of each device 1. Data exchange between two or more than two devices 1 is preferably possible. Data collected and generated by devices 1 can be evaluated by receiving device 18, in particular a computer unit. In case actuator means 24, like watering units or the like, are present on said field, these actuator means 24 can be controlled respectively operated in dependency of said data collected or generated by devices 1. Commands for operating said actuator units 24 are preferably provided by receiving means 18.

Thus, the present invention refers to a device 1 for arrangement underneath one or multiple layers 2, 4, 6 of material. Said device 1 preferably comprises at least a detection unit 8 for detecting of at least one parameter of the material, a communication unit 10 for transmitting data representing the detected parameter through the one or multiple layers 2, 4, 6 of material, wherein the communication unit 10 comprises a RF circuit 12 and an antenna 14, an envelope element 16 for surrounding of at least the communication unit 10, wherein the envelope unit 16 comprises a material composition, wherein the material composition at least comprises a polymer material, a ceramic material and cellulose, wherein the ratio (in wt%) of these materials is selected in such a manner to set up a predefined dielectric property.

### List of reference numbers

- 1: device
- 2: first layer
- 4: second layer
- 6: third layer
- 8: detection unit
- 10: communication unit
- 12: RF circuit
- 14: antenna
- 16: envelope unit
- 18: receiving means
- 19: Signals
- 20: living being
- 22: soil
- 24: actuator means

## Claims

1. Device (1) for arrangement underneath one or multiple layers (2, 4, 6) of material,
at least comprising
a detection unit (8) for detecting of at least one parameter of the material,
a communication unit (10) for transmitting data representing the detected parameter through the one or multiple layers (2, 4, 6) of material,
wherein the communication unit (10) comprises a RF circuit (12) and an antenna (14), an envelope element (16) for surrounding of at least the communication unit (10), wherein the envelope unit (16) comprises a material composition, wherein the material composition at least comprises a polymer material, a ceramic material and cellulose, wherein the ratio (in wt%) of these materials is selected in such a manner to set up a predefined dielectric property.

2. Device according to claim 1,
**characterized in that**
the material composition comprises in wt% more polymer material than the sum of wt% of ceramic material and cellulose.

3. Device according to claim 1 or claim 2,
**characterized in that**
the material composition comprises in wt% more ceramic material than cellulose.

4. Device according to claim 2 or 3,
**characterized in that**
the material composition further comprises at least dye molecules and/or rubber materials, preferred dye molecules are ruthenium and/or perylen and a preferred rubber material is NTN.

5. Device according to claim 2 or 3,
**characterized in that**
the ceramic material and cellulose are provided as particles having a size between 0,1nm and 10*µ*m, in particular between 1 nm and 1*µ*m.

6. Device according to any or the before mentioned claims,
**characterized in that**
the ceramic material and cellulose are provided as powder and mixed homogenously and wherein the ceramic material and cellulose are both capsuled by the polymer material.

7. Device according to any or the before mentioned claims 1 to 4,
**characterized in that**
the material composition forms at least two layers, wherein one layer consists of polymer material and wherein another layer consists of the ceramic material and cellulose, wherein the ceramic material and cellulose are arranged inside a viscose carrier material, in particular a further polymer material.

8. Method for manufacturing of a device (1) according to any of the proceeding claims, at least comprising the steps:
providing a detection unit (8) for detecting of at least one parameter of the material,
and providing a communication unit (10) for transmitting data representing the detected parameter through the one or multiple layers of material,
wherein the communication unit (10) comprises a RF circuit (12) and an antenna (14), and
forming an envelope element (16) for surrounding the detection unit (8) and the communication unit (10),
wherein the envelope unit (16) comprises a material composition, wherein the material composition at least comprises a polymer material, a ceramic material and cellulose, wherein the ratio (in wt%) of these materials is selected in such a manner to set up a predefined dielectric property.

9. Method for supervising of material properties,
at least comprising the steps:
arranging a device (1) according to the one of the before mentioned claims 1 to 7 underneath a predefined material,
wherein the predefined material and the envelope unit (16) are having at least one matching dielectric property,
detecting of at least one parameter with the detection unit (8) and
transmitting data representing the detected parameter through the material layer (2) or material layers (2, 4, 6),
receiving said data by means of a receiving means (18).

10. Method according to claim 9,
**characterized in that**
the device (18) is implanted inside a living being (20).

11. Method according to claim 10,
**characterized in that**
the detection unit (8) is equipped to detect vital functions and/or composition changes of gaseous or liquid or solid substances inside the living being (18).

12. Method according to claim 9,
**characterized in that**
the device (1) is arranged inside soil (22).

13. Method according to claim 12,
**characterized in that**
the detection unit (8) is equipped to detect composition changes of gaseous or liquid or solid substances inside the soil (22), in particular temperature and/or humidity and/or nutrient content.

14. Method according to claims 9 to 13,
**characterized in that**,
the operating frequency of the RF-circuit (12) is set-up in dependency of material properties of the material layer (2) or material layers (4, 6) through which the data has to be transmitted.

15. Wireless mesh network for supervising of an agriculture system
at least comprising
a plurality of devices (1) according to claims 1 to 7,
at least one receiving means (18) for receiving transmitted data
preferably comprising
actuator means (24), in particular watering system,
wherein said actuator means (24) are coupled with said mesh network, for treating said agricultural system, in particular watering, in dependency of the received data.
